# EUROPÄISCHE PATENTSCHRIFT

(11) **EP 1 101 762 B1**
(45) Veröffentlichungstag und Bekanntmachung des Hinweises auf die Patenterteilung: **26.02.2003**
(21) Anmeldenummer: 00124005.0
(22) Anmeldetag: 04.11.2000
(51) Int. Cl.: C07D 317/40, C07D 317/42

(54) **Verfahren zur Herstellung von Vinylencarbonat und dessen Verwendung**
Process for preparation of vinylene carbonate and its use
Procédé pour le préparation de carbonate de vinyléne et son utilisation

(30) Priorität: 19.11.1999 DE 19955944
(43) Veröffentlichungstag der Anmeldung: 23.05.2001
(73) Patentinhaber: MERCK PATENT GmbH, 64293 Darmstadt (DE)
(72) Erfinder: Seifert, Bernhard, 64372 Ober-Ramstadt (DE); Becker, Sylvia, 64342 Seeheim-Jugenheim (DE); Neuschütz, Mark, Dr., 64285 Darmstadt (DE)

(56) Entgegenhaltungen:
- JP-A- 11 180 974
- JP-A- 2000 026 449
- JP-A- 2000 138 071
- US-A- 3 457 279
- CHEMICAL ABSTRACTS, vol. 91, no. 3, 16. Juli 1979 (1979-07-16) Columbus, Ohio, US; abstract no. 21015c, BASSIGNANI L ET AL: "Syntheses of DL-serine and of precursors thereof (glycolaldehyde and "masked" glycolaldehydes)" Seite 707; Spalte 1; XP002901571 & CHEM BER, Bd. 112, Nr. 1, 1979, Seiten 148-160,
- CHEMICAL ABSTRACTS, vol. 90, no. 7, Februar 1979 (1979-02) Columbus, Ohio, US; abstract no. 54350r, DAUB J ET AL: "(C6H6)n-hydrocarbons. 5. Specific reactions at the bicyclo[2.2.2]octadiene part of hexacyclo[9.3.2.24,7.02,9.03,8.010.12]octa deca-5,13,15,17-tetraene" Seite 559; Spalte 1; XP002901572 & CHEM BER, Bd. 111, Nr. 8, 1978, Seiten 2877-2890,
- CHEMICAL ABSTRACTS, vol. 85, no. 11, 13. September 1976 (1976-09-13) Columbus, Ohio, US; abstract no. 77438m, SHAPORO A L ET AL: "Thermocatalytic reactions of chloroethylene carbonates" Seite 490; Spalte 2; XP002901573 & V SB ALKILENKARBONATY, 1975, Seiten 109-122,

## Beschreibung

Die vorliegende Erfindung betrifft ein Verfahren zur Herstellung von Vinylencarbonat sowie die Verwendung des hergestellten Vinylencarbonats, beispielsweise als Additiv für Lithiumionen-Batterien, als Lackkomponente oder als Monomer zur Herstellung von Polyvinylencarbonat.

Aus J. Am. Chem. Soc., 77, 3789-3793 (1955) ist ein Verfahren zur Herstellung von Vinylencarbonat bekannt, bei dem in einem ersten Syntheseschritt Monochlorethylencarbonat durch Chlorieren von Ethylencarbonat hergestellt wird. In einem zweiten Schritt wird eine Lösung von Monochlorethylencarbonat in Ether mit Triethylamin über Nacht unter Rückfluß umgesetzt, um durch Eliminierung von Chlorwasserstoff Vinylencarbonat zu erhalten. Nach Entfernung des Ethers und Destillation wird hierbei Roh-Vinylencarbonat in einer Ausbeute von 59% erhalten, das durch weitere Rektifizierung gereinigt wird. Nachteilig bei diesem Verfahren sind somit die langen Umsetzungszeiten, die relativ aufwendige Aufarbeitung des Reaktionsproduktes zur Entfernung unerwünschter Komponenten, wie Lösungsmittel, sowie die relativ geringe Ausbeute an erwünschtem Produkt.

Der vorliegenden Erfindung liegt daher die Aufgabe zugrunde, ein Verfahren anzugeben, das es in einfacher und wirtschaftlicher Weise ermöglicht. Vinylencarbonat mit hoher Ausbeute herzustellen.

Diese Aufgabe wird durch ein Verfahren gemäß Anspruch 1 gelöst. Vorteilhafte bzw. bevorzugte Ausgestaltungen dieses Verfahrens sind in den Unteransprüchen angegeben.

Gegenstand der Erfindung ist somit ein Verfahren zur Herstellung von Vinylencarbonat der Formel (I) 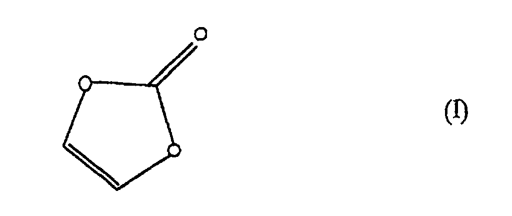 durch Umsetzung eines Monohalogenethylencarbonats der Formel (II) 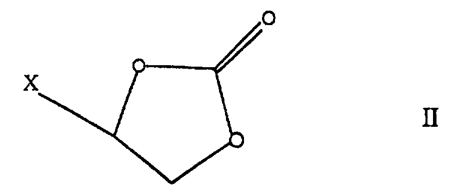 worin X ein Halogenatom bedeutet, mit einem Dehydrohalogenierungsmittel bei erhöhter Temperatur in Gegenwart eines organischen Lösungsmittels, das dadurch gekennzeichnet ist, daß als organisches Lösungsmittel Ethylencarbonat eingesetzt wird.

Beim erfindungsgemäßen Verfahren wird anstelle des bei dem herkömmlichen Verfahren verwendeten Ethers Ethylencarbonat als Lösungsmittel bei der Dehydrohalogenierungsreaktion eingesetzt. Dadurch wird die Zahl der im Reaktionsgemisch enthaltenen, störenden Verbindungen reduziert und somit die Aufreinigung des Reaktionsgemisches erleichtert. Ferner werden beim erfindungsgemäßen Verfahren gegenüber dem bekannten Verfahren deutlich höhere Ausbeuten erzielt. Für bestimmte Anwendungen, beispielsweise als Lösungsmittel für nichtwäßrige Elektrolyte bei Lithiumionen-Batterien, ist es nicht notwendig, das im Reaktionsgemisch enthaltene Ethylencarbonat abzutrennen, vielmehr ist eine solche Vinylencarbonat-Ethylencarbonat-Mischung für diesen Einsatz geradezu erwünscht.

Bei Versuchen hat sich gezeigt. daß Vinylencarbonat stark temperaturempfindhch ist und sich bei Temperaturen oberhalb 60°C innerhalb von Stunden und oberhalb von 80°C sich sogar innerhalb von Minuten zersetzen kann. In der Regel verlaufen jedoch Eliminierungsreaktionen bei höheren Temperaturen mit höheren Ausbeuten. Erfindungsgemäß hat sich gezeigt, daß die hier vorliegende Dehydrohalogenierungsreaktion günstigerweise bei Temperaturen im Bereich von 40-80°C. vorzugsweise bei etwa 60°C, durchgeführt werden kann. Die Umsetzung kann hierbei innerhalb eines Zeitraums von 1-4 Stunden, vorzugsweise etwa 2 Stunden vervollständigt werden. Unter solchen Reaktionsbedingungen liegt die Ausbeute an Roh-Vinylencarbonat üblicherweise bei über 80%.

Für das erfindungsgemäße Verfahren können übliche Dehydrohalogenierungsmittel eingesetzt werden, beispielsweise Alkalilaugen, Amine. Alkylamide oder heterocyclische Stickstoffverbindungen. Vorzugsweise werden Trialkylamine, insbesondere bevorzugt Triethylamin, eingesetzt.

Besonders günstig verläuft das erfindungsgemäße Verfahren unter Verwendung von Monochlorethylencarbonat als Monohalogenethylencarbonat der obigen Formel (II).

Besonders vorteilhaft ist weiterhin die Verwendung einer Schutzgasatmosphäre für die erfindungsgemäße Umsetzung, um Zersetzungsreaktionen zu vermeiden. Als Schutzgase eignen sich beispielsweise Stickstoff oder Edelgase, wie Argon. Damit kann die Verwendung eines üblicherweise eingesetzten Stabilisators für das als Reaktionsprodukt erhaltene Vinylencarbonat entfallen.

Für eine vollständige und gleichmäßige Umsetzung ist es weiterhin vorteilhaft, für eine gute Durchmischung der Reaktionskomponenten zu sorgen.

Bei den erfindungsgemäß als Ausgangsverbindungen eingesetzten Monohalogenethylencarbonaten handelt es sich um bekannte Verbindungen, die beispielsweise durch photochemische Halogenierung oder durch Azoisobutyronitril (AIBN) initiierte Halogenierung von Ethylencarbonat mit beispielsweise Sulfurylchlorid hergestellt werden können. Restmengen an AIBN oder Sulfurylchlorid im Monohalogenethylencarbonat können hierbei zugelassen werden. Vorhandene Restmengen an Sulfurylchlorid können beim erfindungsgemäßen Verfahren beispielsweise durch Verwendung eines entsprechenden Überschusses an Dehydrohalogenierungsmittel, wie Triethylamin, beseitigt werden.

Während beim herkömmlichen Verfahren die Aufreinigung des erhaltenen Roh-Vinylencarbonats durch einfache Destillation erfolgt, hat sich gemäß der Erfindung gezeigt, daß hierbei unerwünschte Ausbeuteverringerungen auftreten können. Vorzugsweise wird daher erfindungsgemäß ein Aufreinigungsverfahren eingesetzt, das eine möglichst kurze Verweilzeit von Vinylencarbonat bei der entsprechenden Verdampfungstemperatur gewährleistet. Dies gelingt beispielsweise durch eine Vakuumdestillation in einem Dünnschichtverdampfer bei Badtemperaturen von etwa 100°C und einem Druck von etwa 5 mbar. Hierdurch gelingt es. direkt aus dem Reaktionsprodukt der Dehydrohalogenierung Vinylencarbonat in einer Ausbeute von etwa mindestens 75% als farbloses Produkt zu erhalten.

Das beim erfindungsgemäßen Verfahren hergestellte Vinylencarbonat kann ohne abtrennung des ethylencarbonats für verschiedene Anwendungen eingesetzt werden, beispielsweise als Additiv für Lithtumionen-Batterien, etwa als Lösungsmittel für nichtwäßrige Elektrolyte, als Lackkomponente oder als Monomer zur Herstellung von Polyvinylencarbonat. Bei der zuletzt genannten Polymerisation können hochmolekulargewichtige, farblose Polymere erhalten werden, die durch eine anschließende Hydrolysereaktion wasserlösliche Polymere ergeben.

Die Erfindung wird durch die nachfolgenden Beispiele näher erläutert.

### Beispiel 1

Eine mit KPG-Rührer. Rührmotor. Intensivkühler, Tropftrichter und Sumpfthermometer ausgestattete 250 ml Doppelmantel-Vierhalsapparatur wird mit Argon gespült. Dann werden unter anhaltender Spülung mit Argon 0,420 Mol Chlorethylencarbonat und 84 ml Ethylencarbonat (wasserfrei) eingebracht. Mittels Heizbad wird auf eine Innentemperatur von 57,6°C erwärmt. Unter Rühren werden dann im Verlaufe von 25 Minuten 0,630 Mol Triethylamin über einen Tropftrichter zugetropft, wobei die Innentemperatur zwischen 56 und 59°C gehalten wird. Nach vollständiger Zugabe des Triethylamins wird das Reaktionsgemisch 1 Stunde bei etwa 60°C gerührt. Danach wird überschüssiges Triethylamin am Rotationsverdampfer bei einer Badtemperatur von 40°C und einem Druck von 150 mbar abdestilliert. Die in der Vinylencarbonat-Rohmischung enthaltene Menge an Vinylencarbonat beträgt 77,2% der Theorie.

### Vergleichsbeispiel 1

Gemäß dem in J. Am. Chem. Soc. 77, 3789-3793 (1955) beschriebenen Verfahren wird Vinylencarbonat hergestellt. Hierzu wird die in Beispiel 1 beschriebene Apparatur mit Argon gespült. Dann werden unter Spülung mit Argon 0.280 Mol Chlorethylencarbonat und 33,4 ml tert.-Butylmethyl-ether (reinst) in die Apparatur eingebracht und die Mischung mittels Heizbad auf 37,8°C erwärmt. Im Verlaufe von 50 Minuten werden dann unter Rühren über einen Tropftrichter 0.350 Mol Triethylamin zugetropft, wobei die Innentemperatur zwischen 37 und 40°C gehalten wird. Danach wird das Reaktionsgemisch unter Rühren 50 Minuten bei etwa 40°C gehalten. Die in der Vinylencarbonat-Rohmischung enthaltene Menge an Vinylencarbonat beträgt nur 26,6% der Theorie.

### Beispiel 2

Die in Beispiel 1 erhaltene Vinylencarbonat-Rohmischung wird durch eine Vakuumdestillation in einem Dünnschichtverdampfer (Innendurchmesser: 40 mm, Rotorlänge: 25 cm) aufgereinigt. Die Badtemperatur beträgt etwa 100°C und der Druck etwa 5 mbar. Bei einer Einspeisungsgeschwindigkeit von ca. 3 ml/min wird nach ca. 70 Minuten ein klares, schwach gelbliches, ölartiges Destillat erhalten. Die Ausbeute an gereinigtem Vinylencarbonat beträgt hierbei 73,3%.

## Patentansprüche

1. Verfahren zur Herstellung von Vinylencarbonat der Formel (I) 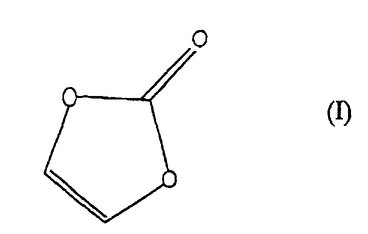 durch Umsetzung eines Monohalogenethylencarbonats der Formel (II) 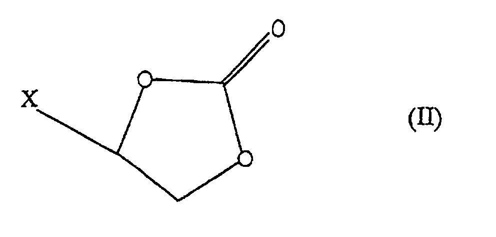 worin X ein Halogenatom bedeutet, mit einem Dehydrohalogenierungsmittel bei erhöhter Temperatur in Gegenwart eines organischen Lösungsmittels, **dadurch gekennzeichnet, daß** als organisches Lösungsmittel Ethylencarbonat eingesetzt wird.

2. Verfahren nach Anspruch 1, **dadurch gekennzeichnet, daß** die Umsetzung bei Temperaturen im Bereich von 40 - 80°C, vorzugsweise etwa 60°C, durchgeführt wird.

3. Verfahren nach Anspruch 1 und/oder 2, **dadurch gekennzeichnet, daß** die Umsetzung während eines Zeitraums von 1 bis 4 Stunden, vorzugsweise etwa 2 Stunden, durchgeführt wird.

4. Verfahren nach mindestens einem der Ansprüche 1-3, **dadurch gekennzeichnet, daß** das Dehydrohalogenierungsmittel aus Alkalilaugen. Aminen. Alkylamiden oder heterocyclischen Stickstoffverbindungen ausgewählt wird.

5. Verfahren nach mindestens einem der Ansprüche 1-4, **dadurch gekennzeichnet, daß** als Dehydrohalogenierungsmittel ein Trialkylamin. vorzugsweise Triethylamin, verwendet wird.

6. Verfahren nach mindestens einem der Ansprüche 1-5, dadurch ge**gekennzeichnet, daß** als Monohalogenethylencarbonat Monochlorethylencarbonat eingesetzt wird.

7. Verfahren nach mindestens einem der Ansprüche 1-6, dadurch ge**gekennzeichnet, daß** die Umsetzung unter einer Schutzgasatmosphäre durchgeführt wird.

8. Verfahren nach mindestens einem der Ansprüche 1-7, dadurch ge**gekennzeichnet, daß** das Vinylencarbonat aus der Reaktionsmischung durch Destillation abgetrennt wird.

9. Verfahren nach Anspruch 8, **dadurch gekennzeichnet, daß** die Abtrennung des Vinylencarbonats durch Vakuumdestillation in einem Dünnschichtverdampfer erfolgt.

10. Verwendung des nach dem Verfahren gemäß mindestens einem der Ansprüche 1-9 hergestellten Vinylencarbonats ohne Abtrennung des Ethylencarbonats als Additiv für Lithiumionen-Batterien, als Lackkomponente oder als Monomer zur Herstellung von Polyvinylencarbonat.

## Claims

1. Process for the preparation of vinylene carbonate of the formula (I) 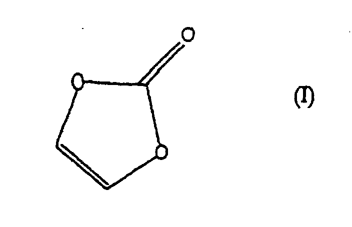 by reacting a monohaloethylene carbonate of the formula (II) 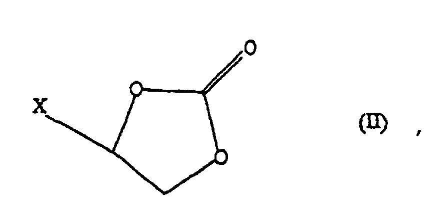 in which X is a halogen atom, with a dehydrohalogenating agent at elevated temperature in the presence of an organic solvent, **characterized in that** the organic solvent employed is ethylene carbonate.

2. Process according to Claim 1, **characterized in that** the reaction is carried out at temperatures in the range 40 - 80°C, preferably about 60°C.

3. Process according to Claim 1 and/or 2, **characterized in that** the reaction is carried out for a period of from 1 to 4 hours, preferably about 2 hours.

4. Process according to at least one of Claims 1-3, **characterized in that** the dehydrohalogenating agent is selected from alkali metal hydroxide solutions, amines, alkylamides and heterocyclic nitrogen compounds.

5. Process according to at least one of Claims 1-4, **characterized in that** the dehydrohalogenating agent used is a trialkylamine, preferably triethylamine.

6. Process according to at least one of Claims 1-5, **characterized in that** the monohaloethylene carbonate employed is monochloroethylene carbonate.

7. Process according to at least one of Claims 1-6, **characterized in that** the reaction is carried out under a protective-gas atmosphere.

8. Process according to at least one of Claims 1-7, **characterized in that** the vinylene carbonate is separated off from the reaction mixture by distillation.

9. Process according to Claim 8, **characterized in that** the separation of the vinylene carbonate is carried out by vacuum distillation in a thin-film evaporator.

10. Use of the vinylene carbonate prepared by the process according to at least one of Claims 1-9, without the separation of the ethylene carbonate, as an additive for lithium ion batteries, as a component of surface coatings or as a monomer for the preparation of polyvinylene carbonate.

## Revendications

1. Préparation pour la préparation du carbonate de vinylène de formule (I) 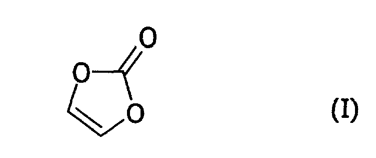 par réaction d'un carbonate de monohalogénoéthylène de formule (II) 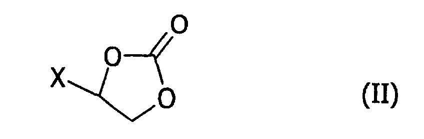 où X représente un atome d'halogène, avec un agent de déshydrohalogénation à une température augmentée en présence d'un solvant organique, **caractérisé en ce que** l'on utilise du carbonate d'éthylène comme solvant organique.

2. Procédé selon la revendication 1, **caractérisé en ce que** l'on effectue la réaction à des températures comprises dans une plage allant de 40 à 80°C, de préférence à environ 60°C.

3. Procédé selon la revendication 1 et/ou 2, **caractérisé en ce que** l'on effectue la réaction pendant un intervalle de temps allant de 1 à 4 h, de préférence pendant environ 2 h.

4. Procédé selon au moins l'une des revendications 1 à 3, **caractérisé en ce que** l'agent de déshydrohalogénation est choisi dans le groupe constitué par les lessives alcalines, les amines, les alkylamides ou les composés azotés hétérocycliques.

5. Procédé selon au moins l'une des revendications 1 à 4, **caractérisé en ce que** l'on utilise comme agent de déshydrohalogénation une trialkylamine, de préférence la triéthylamine.

6. Procédé selon au moins l'une des revendications 1 à 5, **caractérisé en ce que** l'on utilise comme carbonate de monohalogénoéthylène le carbonate de monochloroéthylène.

7. Procédé selon au moins l'une des revendications 1 à 6, **caractérisé en ce que** l'on effectue la réaction sous une atmosphère de gaz protecteur.

8. Procédé selon au moins l'une des revendications 1 à 7, **caractérisé en ce que** l'on sépare par distillation le carbonate de vinylène du mélange réactionnel.

9. Procédé selon la revendication 8, **caractérisé en ce que** la séparation du carbonate de vinylène s'effectue par distillation sous vide dans un évaporateur à couche mince.

10. Utilisation du carbonate de vinylène préparé par le procédé selon au moins l'une des revendications 1 à 9, sans séparation du carbonate d'éthylène, comme additif pour les batteries aux ions lithium, comme composition de vernis ou comme monomère pour la préparation de carbonate de polyvinylène.
